Europäisches Patentamt

European Patent Office

Office européen des brevets

(11) Publication number: **0 264 979**
**A1**

## (12) EUROPEAN PATENT APPLICATION

(21) Application number: 87201657.1

(22) Date of filing: 01.09.87

(51) Int. Cl.4: **C12N 15/00** , **A61K 39/215** , **A61K 37/02** , **C07K 13/00**

(30) Priority: 05.09.86 NL 8602244

(43) Date of publication of application:
27.04.88 Bulletin 88/17

(84) Designated Contracting States:
AT BE CH DE ES FR GB GR IT LI LU NL SE

(71) Applicant: **DUPHAR INTERNATIONAL RESEARCH B.V**
C.J. van Houtenlaan 36
NL-1381 CP Weesp(NL)

(72) Inventor: **de Groot, Raoul J.**
c/o OCTROOIBUREAU ZOAN B.V. P.O. Box 140
NL-1380 AC Weesp(NL)
Inventor: **Spaan, Wilhelmus J.M.**
c/o OCTROOIBUREAU ZOAN B.V. P.O. Box 140
NL-1380 AC Weesp(NL)
Inventor: **van der Zeijst, Bernardus A.M.**
c/o OCTROOIBUREAU ZOAN B.V. P.O. Box 140
NL-1380 AC Weesp(NL)

(74) Representative: **Muis, Maarten et al**
**OCTROOIBUREAU ZOAN B.V. P.O. Box 140**
**NL-1380 AC Weesp(NL)**

(54) New antigenically active proteins and peptides, and feline infectious peritonitis virus (FIPV) vaccines.

(57) The invention relates to new antigenic proteins which can be used to simulate the immunity of cats (Felidae) against infectious peritonitis virus (FIPV), and to fragments or derivatives thereof. The invention also relates to vaccines which comprise such antigenic proteins or peptides.

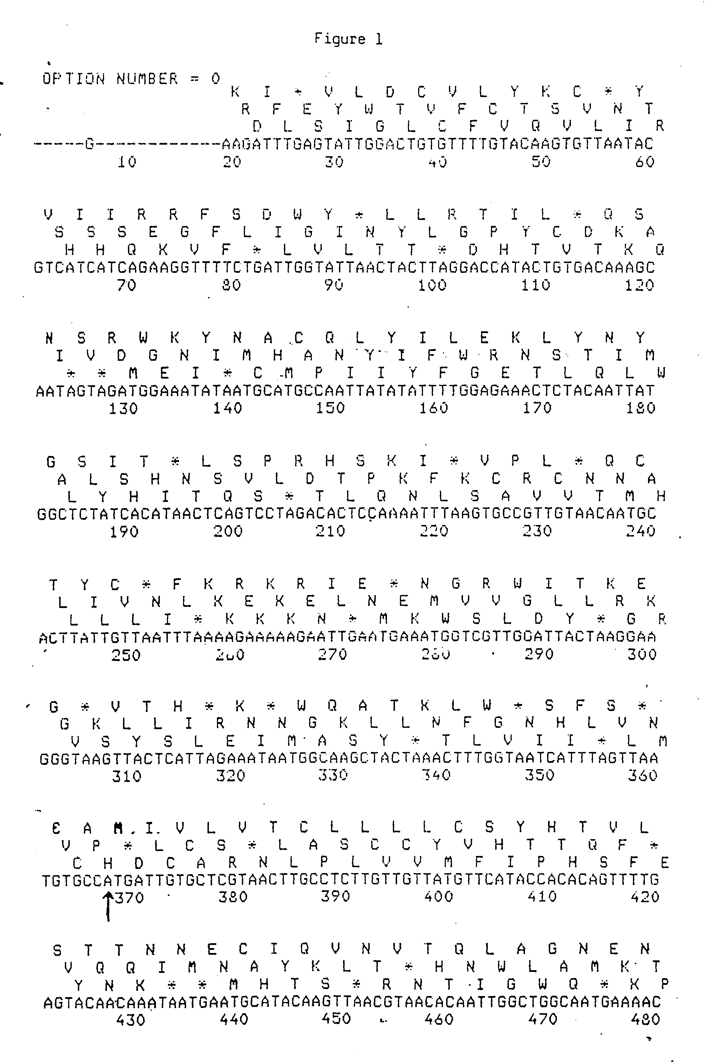

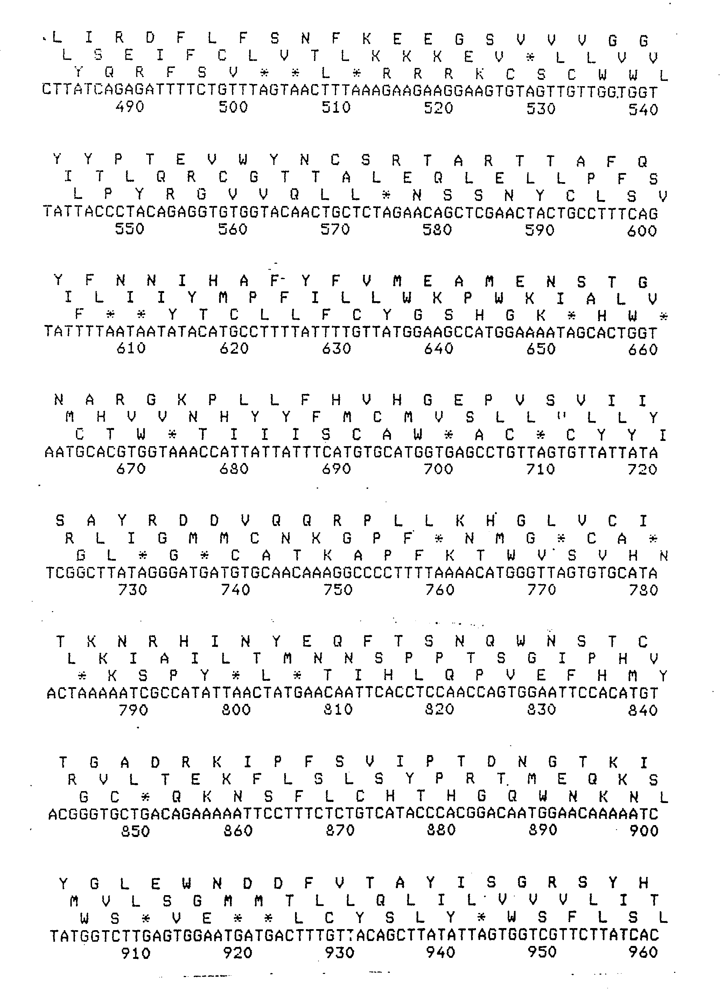

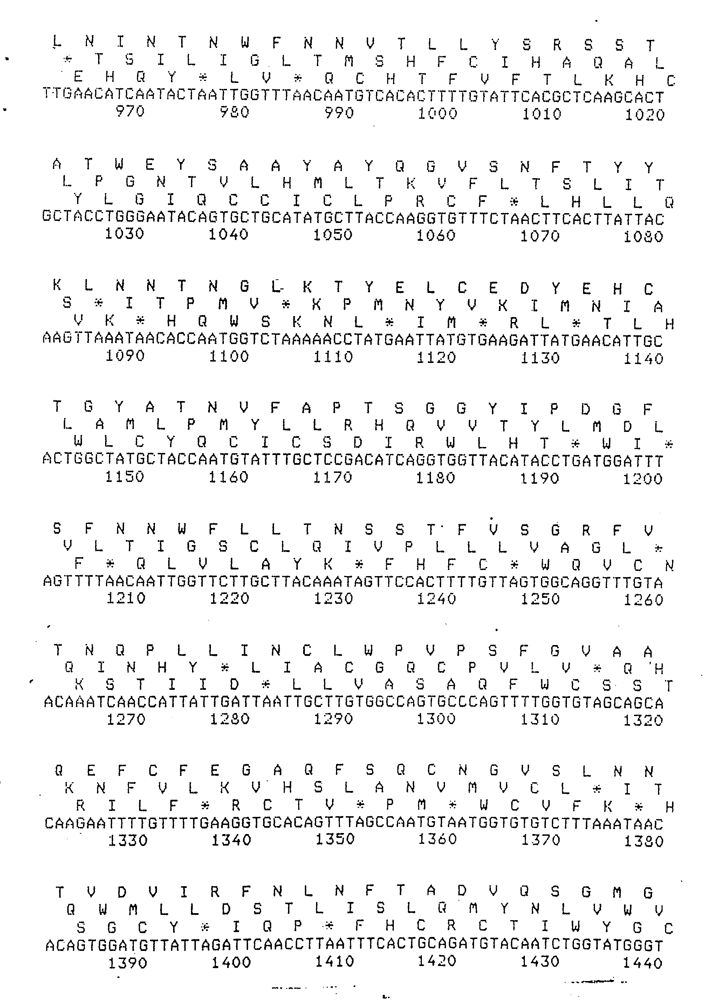

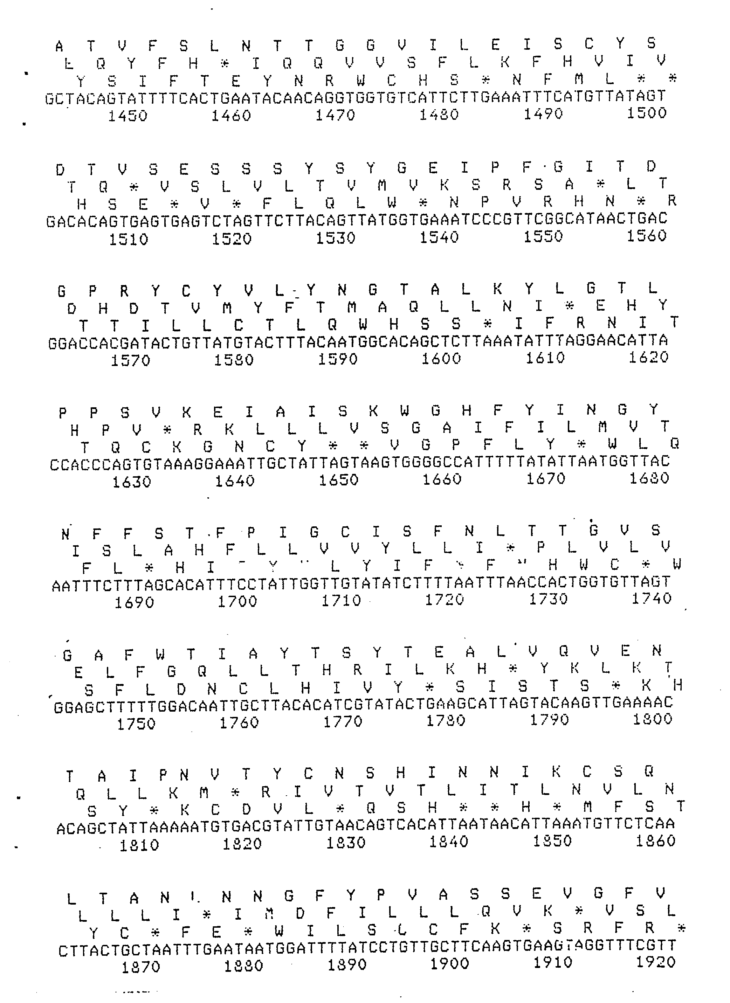

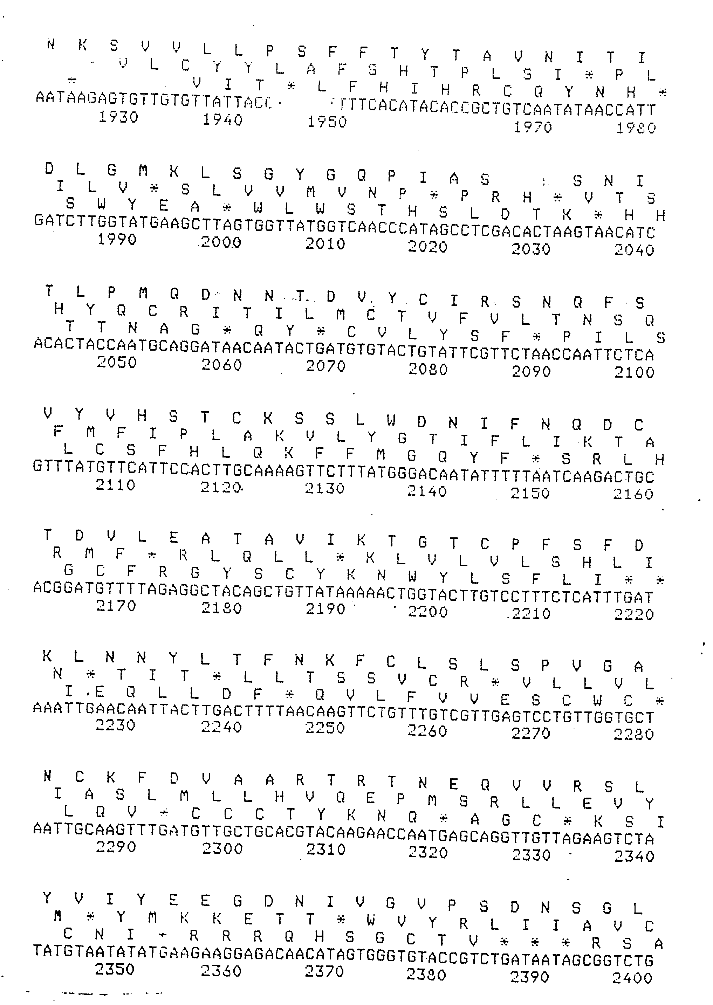

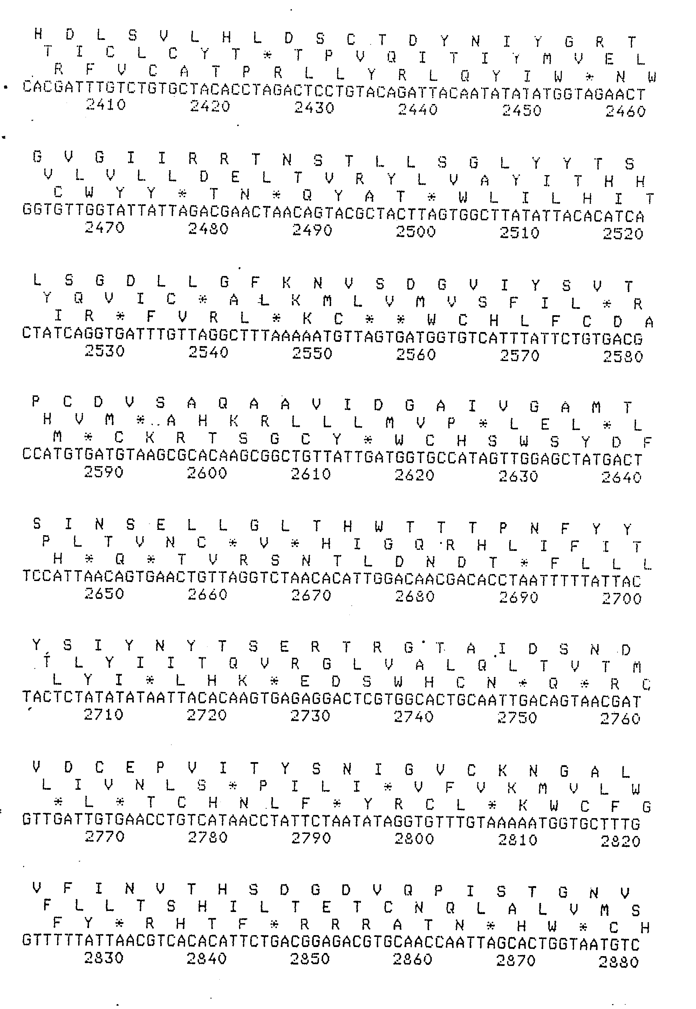

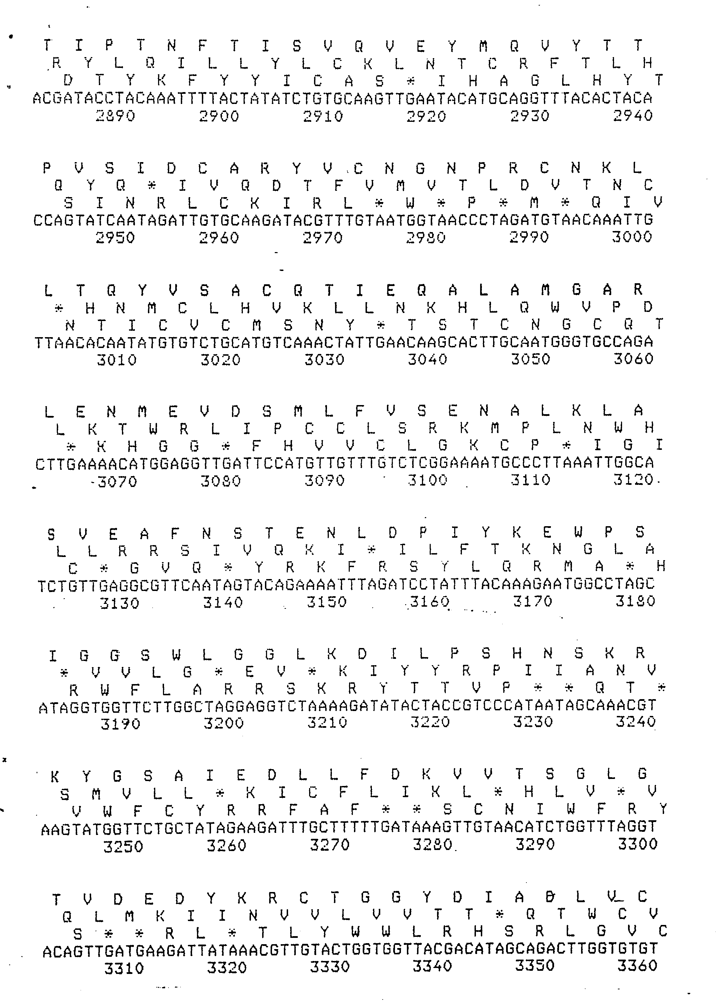

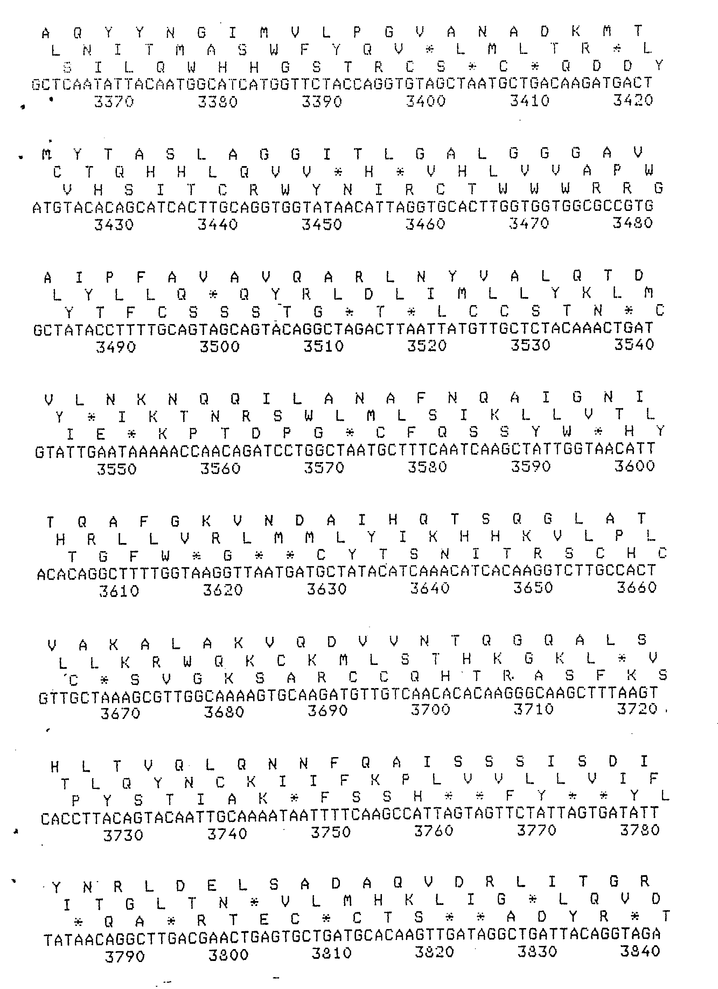

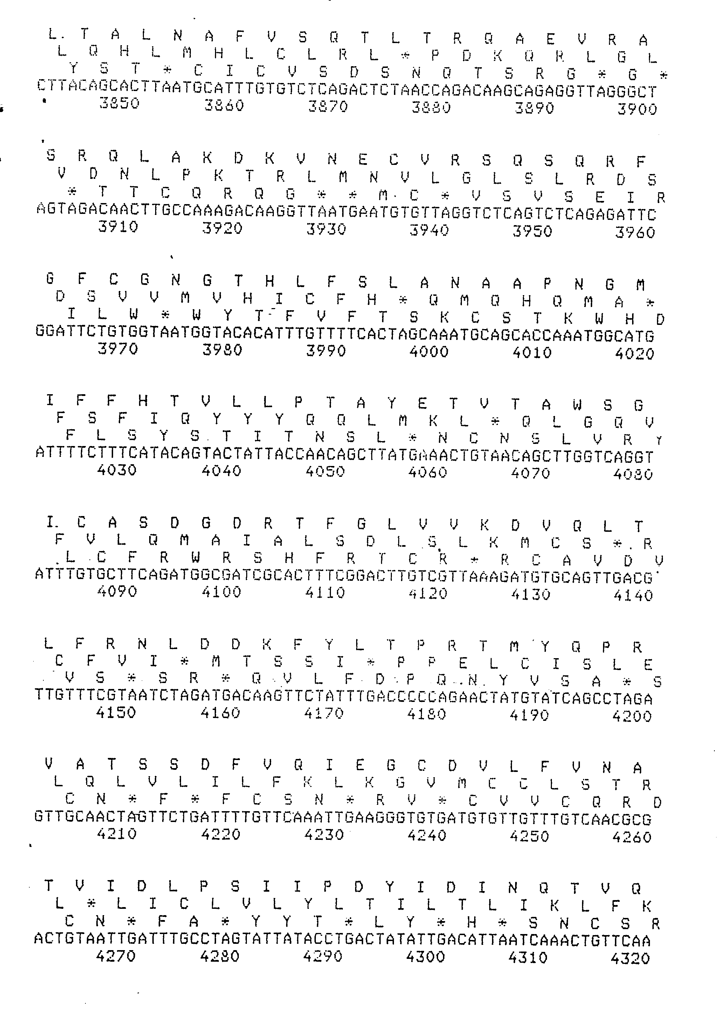

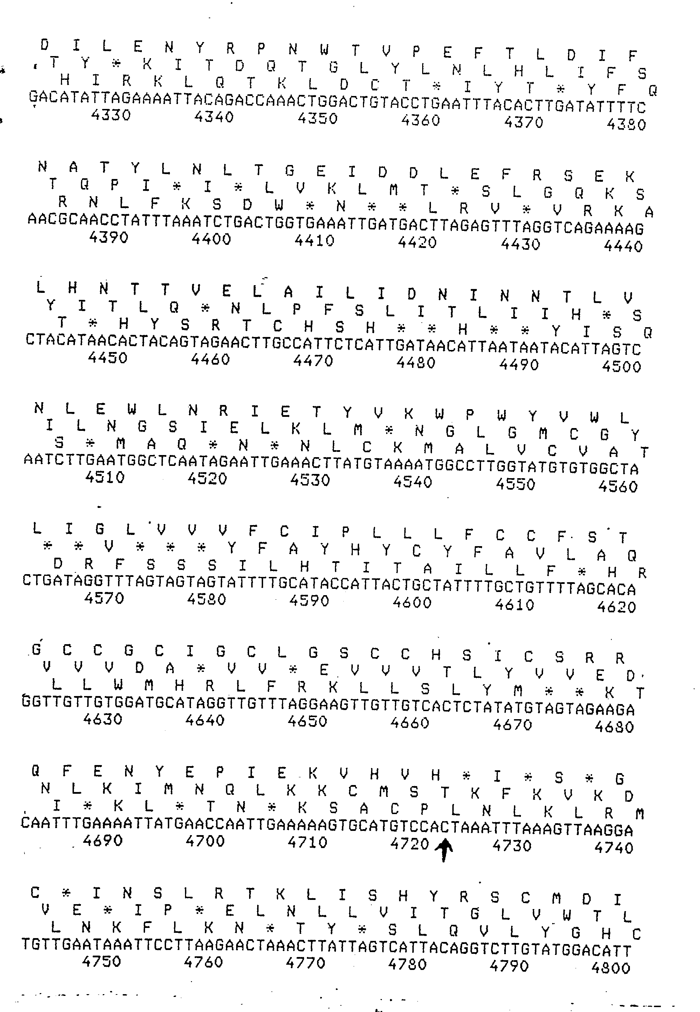

The nucleotide sequence and amine acid sequence of a gene coding for protein of an FIPV-strain are indicated in figure 1.

## New antigenically active proteins and peptides, and feline infectious peritonitis virus (FIPV) vaccines.

The invention relates to a new antigenic protein which can stimulate the immunicity of cats (Felidae) against feline infectious peritonitis virus (FIPV), and to fragments or derivatives thereof. The invention further relates to vaccines which comprise such an antigenic protein or peptide.

Feline infectious peritonitis is an infectious disease which usually is lethal. The disease is caused by a virus, the feline infectious peritonitis (FIP)-virus. The clinical symptoms of naturally obtained FIP are not very characteristic initially. An animal with FIP usually shows anorexia, anaemia, and in most cases an elevated temperature and complete apathy. In classical FIP-cases, said symptoms are accompanied by a gradual swelling of the belly, which, in combination with a progressing emaciation, results in a dehydrated animal having a swollen undulating belly. When these symptoms are observed, death usually follows within 1-8 weeks. Therapeutic measures have so far proved little effective and the prognosis for the disease is particularly poor. Prophilactic measures cannot be taken either, since the pathogenesis and the way in which the virus is transferred have not yet been elucidated. The development of an active vaccine hence is urgently necessary. Via the classical route one has so far not been able to prepare an active vaccine against FIP.

FIPV belongs to the corona viruses. Corona viruses are positive single-stranded RNA-viruses with a lipide envelope. The genome RNA has a length of approximately 20,000 bases. The virus particle (virion) comprises three important proteins namely the peplomeric protein (S), the nucleocapside protein (N), and the matrix protein (M).

The characteristic protrusions at the surface of the virion are formed by the peplemeric protein (S). Each peplomer consists of the two glycopolypeptides $S_1$ and $S_2$. The $S_2$ protein is fixed to the membrane, and the $S_1$ protein is coupled via the $S_2$ protein.

According to the present invention, the nucleotide sequence and the amino acid sequence of the FIPV strain 79-1146 (McKeirman et al, Feline Practice No. 11, 1981) were determined.

The invention therefore relates to proteins and fragments thereof which comprise at least one of the regions which play a role in the protection against FIP infections.

Theoretically, such fragments may be used as such for vaccinating against FIP. However, it is to be preferred to couple the said fragments to a suitable carrier.

The peptide fragments or proteins according to the invention which comprise at least one antigenically active peptide fragment may be prepared according to methods known for the preparation of peptides and proteins.

First of all, the peptides may be prepared synthetically by means of known techniques starting from separate amino acids or smaller peptide fragments.

The peptides may also be obtained biosynthetically using recombinant DNA techniques and expression systems, for example, by

(a) transformation of host cells with an expression vector which comprises a DNA coding for an (the) antigenic determinant(s) (peptides in general)l

(b) expressing the genome introduced into the expression vector;

(c) harvesting the cell culture, and

(d) isolating the synthesised peptide (protein).

Finally the genetic information coding for the peptide(s) and protein(s) according to the invention which comprise at least one of the regions which play a role in the protection against FIP infections. may be introduced in a live recombinant carrier with the aim of expression of said peptide(s) or protein(s) in susceptible host cells and/or host organisms.

The invention therefore also relates to a method of preparing a DNA molecule which codes for peptides according to the invention. Such a method comprises the following steps:

(a) isolating FIP single-stranded RNA;

(b) synthesis of a cDNA strain which is complementary to the RNA strain mentioned in (a); and

(c) removing the RNA molecule and synthesising a second cDNA strain with the first cDNA strain as template.

The double-strained DNA obtained in this manner may be inserted in a manner known per se into an expression vector so that a recombinant expression vector is formed. this vector may be introduced, for example, by transformation, into a suitable host cell.

Also the double-stranded DNA obtained in this manner may be inserted in a manner known per se into a live recombinant carrier.

The invention will now be described in greater detail with reference to the ensuing specific example.

EXAMPLE

Virus and tissue culture

FIPV strain 79-1146 was purified twice by means of end point dilution on fcwf-D cells and was then used for making virus stocks.

(a) Isolation of viral genome RNA

Fcwf-D cells were infected with FPV 79-1146 with a multiplicity of infection of 0.1 $TCID_{50}$. Sixteen hours after infection, the medium was harvested and centrifuged at 1000x g for10 minutes so as to remove cell debris. NaCl and polyethylene glycol (BDH, Poole, England) were added to a final concentration of 2.33% (wt./vol.) and 10% (wt./vol.), respectively. The mixture was centrifuged at 4°C and 10,000x g for 16 hours. The pellet was resuspended in TESV-buffer (0,02 M Tris-Cl, pH 7.4./ 1mM EDTA, 0.1 M NaCl). After centrifugation at 10,000x g for 10 minutes, the virus suspension was provided on a 10% (wt./vol.) sucrose layer which was layered on a 20-50% (wt./vol.) sucrose gradient + |sucrose solution in TESV). The gradient was centrifuged at 4°C and 16,000 rpm fo r16 hours in an SW 27.0 rotor. The virus band was harvested and diluted three .time in TESV.. The virus was pelleted by centrifugation at 4°C and 250,000x g for 90 minutes and then incubated at 37°C for 30 minutes in TESV, 0.5% of SDS, 0.5 mg of proteinase K. The RNA was finally extracted with phenol--chloroform, succeeded by ether extraction and ethanol precipitation. In this manner, approximately 5 μg genome RNA could be obtained from $2 \times 10^{\varepsilon}$ infected cells.

(b) Cloning of FIPV genome RNA

The synthetisis of cDNA was carried out as described by Gubler and Hoffman (Gener 25 - (1983). 263-269). Calf thymus DNA pentamers as ("random") primers and FIPV genome RNA denaturated with methyl mercury hydroxide were used. The double-stranded cDNA was tailed with dC-residues and then ligated in Pstl-digerated dG-tailed pUC9. The recombinant plasmides thus obtained were used for the transformation of E.coli strain JM109 as described by Hanahan (J. Mol, Biol. 166 (1983), 557-580). In this manner a cDNA bank of 400 transformants were obtained. The plasmides comprise inserts having a length up to 6 kb with an average size of 2.5 kb.

(c) Selection of recombinants comprising $E_2$ sequences.

Recombinants with $E_2$-specific sequences could be identified by using the fact that coronaviral RNAs have a 3'coterminal "nested set" structure. Of each messenger the coding information lies on the extreme 5' segment which is absent in the subsequent smaller DNA of the set. Therefore it was possible to select $E_2$-sequences by means of differential hybridisation. Southern blots of recombinant plasmides were hybridised with $^{32}P$ labelled RNA fractions which were enriched for the 9.6 and the 5.2 kb FIPV RNA, respectively. The fractions were obtained by isokinetic sucrose gradient centrifugation of total poly(A)+ RNA from FIPV infected cells. A plasmide pUC-FUPV-III-3 hybridised with the first but not with the last-mentioned probe and was therefore used for further screening of the clone bank.

(d) Characterisation of cloned sequences.

The 5' end of the 9.6 kb RNA-"body" was mapped by means of $S_1$-analysis according to Berk and Sharp (Cell 12 (1977), 721-732). Restriction fragments were separated on agorose gels, isolated by means of NA45-membranes (Schleicher and Schnell) and sub-cloned in M13 vectors. Sequence analysis was carried out according to the dideoxy-method (Sanger et al, Proc. Natl. Acad. Sci, USA 74 (1977), 5463-5467).Both the universal M13-primer and internal synthetic primers (17-mer) were used. The data were analysed by means of a DEC 20/60 computer and the programs of Staden, Nucl. Acid. Res. 10 (1982), 4731-4751.

(e) Nucleotide sequence.

The nucleotide sequence of the peplomeric gene of FIPV strain 79-1146 is shown in Figure 1. In this Figure the three reading frames are shown. The top reading frame is an open reading frame of 4356 nucleotides having a coding capacity for a precursor polypeptide having a molecular weight of 160,470 (1452 amino acids). The beginning and the end of the $E_2$ gene are indicated by arrows.

(f) Amino acid sequence.

The first 18 N-terminal amino acids have a strong hydrophobic character and presumably comprise a cleavable signal peptide. The extreme carboxy-terminal part comprises, as was earlier found for the peplomeric protein of the infectious bronchitis virus (IBV), a region of 20 hydrophobic amino acids, which presumably serves as a transmembrane anchor. The FIPV peplomeric protein has 35 potential glycosylation sites, of which 22 are in the N-terminal part (pos, 1-790) which corresponds to the $S_1$-part of the IBV $E_2$.

## Claims

1. A gene which codes for protein of feline infectious peritonitis virus (FIPV), characterized by the nucleotide sequence as shown in Figure 1.

2. A protein characterized by the amino acid sequence as shown in Figure 1, or a part thereof.

3. An immunogen, characterized in that it comprises a protein or peptide as claimed in Claim 2, coupled or not coupled to a carrier.

4. A vaccine, characterized in that it comprises an immunogen as claimed in Claim 3.

5. A method of preparing a protein or peptide as claimed in Claim 2 characterized in that

(a) the protein or peptide is prepared synthetically in a known manner from separate amino acids and/or by coupling of smaller peptides; or

(b) a host cell is transformed with an expression vector or infected with a live recombinant carrier which comprises a DNA which is coding for a protein or peptide as claimed in Claim 2, and the genome inserted into the expression vector is expressed.

6. A DNA molecule which comprises a nucleotide sequence which codes for a protein or peptide as claimed in Claim 2, or which codes for a polypeptide which comprises a protein or peptide as claimed in Claim 2.

7. A recombinant DNA expression vector or live recombinant carrier which expresses the whole protein or peptide or a part thereof as defined in Claim 2, comprising an operon with initiator sequences and terminator sequences and a nucleotide sequence which codes for the said protein or peptide, the said nucleotide being situated between the initiator sequences and the terminator sequences of the operon.

8. A host cell which comprises a recombinant DNA expression vector and/or a DNA molecule as claimed in Claim 6 or 7.

9. A method of preparing a DNA molecule which codes for a protein or peptide as claimed in Claim 2, characterized in that

(a) single-stranded FIPV-RNA is isolated;

(b) a cDNA strand whichis complementary to the said RNA strand is synthesised;

(c) the RNA strand is removed and a second strand of cDNA is synthesised with the first cDNA strand as template.

Figure 1

```
OPTION NUMBER = 0
                      K  I  *  V  L  D  C  V  L  Y  K  C  *  Y
                        R  F  E  Y  W  T  V  F  C  T  S  V  N  T
                          D  L  S  I  G  L  C  F  V  Q  V  L  I  R
------G---------------AAGATTTGAGTATTGGACTGTGTTTTGTACAAGTGTTAATAC
       10        20        30        40        50        60


    V  I  I  R  R  F  S  D  W  Y  *  L  L  R  T  I  L  *  Q  S
      S  S  S  E  G  F  L  I  G  I  N  Y  L  G  P  Y  C  D  K  A
       H  H  Q  K  V  F  *  L  V  L  T  T  *  D  H  T  V  T  K  Q
    GTCATCATCAGAAGGTTTTCTGATTGGTATTAACTACTTAGGACCATACTGTGACAAAGC
       70        80        90       100       110       120


    N  S  R  W  K  Y  N  A  C  Q  L  Y  I  L  E  K  L  Y  N  Y
      I  V  D  G  N  I  M  H  A  N  Y  I  F  W  R  N  S  T  I  M
       *  *  M  E  I  *  C  M  P  I  I  Y  F  G  E  T  L  Q  L  W
    AATAGTAGATGGAAATATAATGCATGCCAATTATATATTTTGGAGAAACTCTACAATTAT
      130       140       150       160       170       180


    G  S  I  T  *  L  S  P  R  H  S  K  I  *  V  P  L  *  Q  C
      A  L  S  H  N  S  V  L  D  T  P  K  F  K  C  R  C  N  N  A
       L  Y  H  I  T  Q  S  *  T  L  Q  N  L  S  A  V  V  T  M  H
    GGCTCTATCACATAACTCAGTCCTAGACACTCCAAAATTTAAGTGCCGTTGTAACAATGC
      190       200       210       220       230       240


    T  Y  C  *  F  K  R  K  R  I  E  *  N  G  R  W  I  T  K  E
      L  I  V  N  L  K  E  K  E  L  N  E  M  V  V  G  L  L  R  K
       L  L  L  I  *  K  K  K  N  *  M  K  W  S  L  D  Y  *  G  R
    ACTTATTGTTAATTTAAAAGAAAAGAATTGAATGAAATGGTCGTTGGATTACTAAGGAA
      250       260       270       280        290       300


    G  *  V  T  H  *  K  *  W  Q  A  T  K  L  W  *  S  F  S  *
      G  K  L  L  I  R  N  N  G  K  L  L  N  F  G  N  H  L  V  N
       V  S  Y  S  L  E  I  M  A  S  Y  *  T  L  V  I  I  *  L  M
    GGGTAAGTTACTCATTAGAAATAATGGCAAGCTACTAAACTTTGGTAATCATTTAGTTAA
      310       320       330       340       350       360


    E  A  M  I  V  L  V  T  C  L  L  L  L  C  S  Y  H  T  V  L
      V  P  *  L  C  S  *  L  A  S  C  C  Y  V  H  T  T  Q  F  *
       C  H  D  C  A  R  N  L  P  L  V  V  M  F  I  P  H  S  F  E
    TGTGCCATGATTGTGCTCGTAACTTGCCTCTTGTTGTTATGTTCATACCACACAGTTTTG
          ↑370       380       390       400       410       420


    S  T  T  N  N  E  C  I  Q  V  N  V  T  Q  L  A  G  N  E  N
      V  Q  Q  I  M  N  A  Y  K  L  T  *  H  N  W  L  A  M  K  T
       Y  N  K  *  *  M  H  T  S  *  R  N  T  I  G  W  Q  *  K  P
    AGTACAACAAATAATGAATGCATACAAGTTAACGTAACACAATTGGCTGGCAATGAAAAC
      430       440       450       460       470       480
```

DUPHAR INTERNATIONAL RESEARCH B.V.

```
 L  I  R  D  F  L  F  S  N  F  K  E  E  G  S  V  V  V  G  G
  L  S  E  I  F  C  L  V  T  L  K  K  K  E  V  *  L  L  V  V
   Y  Q  R  F  S  V  *  *  L  *  R  R  R  K  C  S  C  W  W  L
CTTATCAGAGATTTTCTGTTTAGTAACTTTAAAGAAGAAGGAAGTGTAGTTGTTGGTGGT
     490       500       510       520       530       540
```

```
 Y  Y  P  T  E  V  W  Y  N  C  S  R  T  A  R  T  T  A  F  Q
  I  T  L  Q  R  C  G  T  T  A  L  E  Q  L  E  L  L  P  F  S
   L  P  Y  R  G  V  V  Q  L  L  *  N  S  S  N  Y  C  L  S  V
TATTACCCTACAGAGGTGTGGTACAACTGCTCTAGAACAGCTCGAACTACTGCCTTTCAG
     550       560       570       580       590       600
```

```
 Y  F  N  N  I  H  A  F  Y  F  V  M  E  A  M  E  N  S  T  G
  I  L  I  I  Y  M  P  F  I  L  L  W  K  P  W  K  I  A  L  V
   F  *  *  Y  T  C  L  L  F  C  Y  G  S  H  G  K  *  H  W  *
TATTTTAATAATATACATGCCTTTTATTTTGTTATGGAAGCCATGGAAAATAGCACTGGT
     610       620       630       640       650       660
```

```
 N  A  R  G  K  P  L  L  F  H  V  H  G  E  P  V  S  V  I  I
  M  H  V  V  N  H  Y  Y  F  M  C  M  V  S  L  L  "  L  L  Y
   C  T  W  *  T  I  I  I  S  C  A  W  *  A  C  *  C  Y  Y  I
AATGCACGTGGTAAACCATTATTATTTCATGTGCATGGTGAGCCTGTTAGTGTTATTATA
     670       680       690       700       710       720
```

```
 S  A  Y  R  D  D  V  Q  Q  R  P  L  L  K  H  G  L  V  C  I
  R  L  I  G  M  M  C  N  K  G  P  F  *  N  M  G  *  C  A  *
   G  L  *  G  *  C  A  T  K  A  P  F  K  T  W  V  S  V  H  N
TCGGCTTATAGGGATGATGTGCAACAAAGGCCCCTTTTAAAACATGGGTTAGTGTGCATA
     730       740       750       760       770       780
```

```
 T  K  N  R  H  I  N  Y  E  Q  F  T  S  N  Q  W  N  S  T  C
  L  K  I  A  I  L  T  M  N  N  S  P  P  T  S  G  I  P  H  V
   *  K  S  P  Y  *  L  *  T  I  H  L  Q  P  V  E  F  H  M  Y
ACTAAAAAATCGCCATATTAACTATGAACAATTCACCTCCAACCAGTGGAATTCCACATGT
     790       800       810       820       830       840
```

```
 T  G  A  D  R  K  I  P  F  S  V  I  P  T  D  N  G  T  K  I
  R  V  L  T  E  K  F  L  S  L  S  Y  P  R  T  M  E  Q  K  S
   G  C  *  Q  K  N  S  F  L  C  H  T  H  G  Q  W  N  K  N  L
ACGGGTGCTGACAGAAAAATTCCTTTCTCTGTCATACCCACGGACAATGGAACAAAAATC
     850       860       870       880       890       900
```

```
 Y  G  L  E  W  N  D  D  F  V  T  A  Y  I  S  G  R  S  Y  H
  M  V  L  S  G  M  M  M  T  L  L  Q  L  I  L  V  V  V  L  I  T
   W  S  *  V  E  *  *  L  C  Y  S  L  Y  *  W  S  F  L  S  L
TATGGTCTTGAGTGGAATGATGACTTTGTTACAGCTTATATTAGTGGTCGTTCTTATCAC
     910       920       930       940       950       960
```

DUPHAR INTERNATIONAL RESEARCH B.V.

```
  L   N   I   N   T   N   W   F   N   N   V   T   L   L   Y   S   R   S   S   T
  *   T   S   I   L   I   G   L   T   M   S   H   F   C   I   H   A   Q   A   L
    E   H   Q   Y   *   L   V   *   Q   C   H   T   F   V   F   T   L   K   H   C
TTGAACATCAATACTAATTGGTTTAACAATGTCACACTTTTGTATTCACGCTCAAGCACT
     970       980       990      1000      1010      1020
```

```
  A   T   W   E   Y   S   A   A   Y   A   Y   Q   G   V   S   N   F   T   Y   Y
  L   P   G   N   T   V   L   H   M   L   T   K   V   F   L   T   S   L   I   T
    Y   L   G   I   Q   C   C   I   C   L   P   R   C   F   *   L   H   L   L   Q
GCTACCTGGGAATACAGTGCTGCATATGCTTACCAAGGTGTTTCTAACTTCACTTATTAC
    1030      1040      1050      1060      1070      1080
```

```
  K   L   N   N   T   N   G   L   K   T   Y   E   L   C   E   D   Y   E   H   C
  S   *   I   T   P   M   V   *   K   P   M   N   Y   V   K   I   M   N   I   A
    V   K   *   H   Q   W   S   K   N   L   *   I   M   *   R   L   *   T   L   H
AAGTTAAATAACACCAATGGTCTAAAAACCTATGAATTATGTGAAGATTATGAACATTGC
    1090      1100      1110      1120      1130      1140
```

```
  T   G   Y   A   T   N   V   F   A   P   T   S   G   G   Y   I   P   D   G   F
  L   A   M   L   P   M   Y   L   L   R   H   Q   V   V   T   Y   L   M   D   L
    W   L   C   Y   Q   C   I   C   S   D   I   R   W   L   H   T   *   W   I   *
ACTGGCTATGCTACCAATGTATTTGCTCCGACATCAGGTGGTTACATACCTGATGGATTT
    1150      1160      1170      1180      1190      1200
```

```
  S   F   N   N   W   F   L   L   T   N   S   S   T   F   V   S   G   R   F   V
  V   L   T   I   G   S   C   L   Q   I   V   P   L   L   L   V   A   G   L   *
    F   *   Q   L   V   L   A   Y   K   *   F   H   F   C   *   W   Q   V   C   N
AGTTTTAACAATTGGTTCTTGCTTACAAATAGTTCCACTTTTGTTAGTGGCAGGTTTGTA
    1210      1220      1230      1240      1250      1260
```

```
  T   N   Q   P   L   L   I   N   C   L   W   P   V   P   S   F   G   V   A   A
  Q   I   N   H   Y   *   L   I   A   C   G   Q   C   P   V   L   V   *   Q   H
    K   S   T   I   I   D   *   L   L   V   A   S   A   Q   F   W   C   S   S   T
ACAAATCAACCATTATTGATTAATTGCTTGTGGCCAGTGCCCAGTTTTGGTGTAGCAGCA
    1270      1280      1290      1300      1310      1320
```

```
  Q   E   F   C   F   E   G   A   Q   F   S   Q   C   N   G   V   S   L   N   N
  K   N   F   V   L   K   V   H   S   L   A   N   V   M   V   C   L   *   I   T
    R   I   L   F   *   R   C   T   V   *   P   M   *   W   C   V   F   K   *   H
CAAGAATTTTGTTTTGAAGGTGCACAGTTTAGCCAATGTAATGGTGTGTCTTTAAATAAC
    1330      1340      1350      1360      1370      1380
```

```
  T   V   D   V   I   R   F   N   L   N   F   T   A   D   V   Q   S   G   M   G
  Q   W   M   L   L   D   S   T   L   I   S   L   Q   M   Y   N   L   V   W   V
    S   G   C   Y   *   I   Q   P   *   F   H   C   R   C   T   I   W   Y   G   C
ACAGTGGATGTTATTAGATTCAACCTTAATTTCACTGCAGATGTACAATCTGGTATGGGT
    1390      1400      1410      1420      1430      1440
```

```
  A  T  V  F  S  L  N  T  T  G  G  V  I  L  E  I  S  C  Y  S
 L  Q  Y  F  H  *  I  Q  Q  V  V  S  F  L  K  F  H  V  I  V
   Y  S  I  F  T  E  Y  N  R  W  C  H  S  *  N  F  M  L  *  *
GCTACAGTATTTTCACTGAATACAACAGGTGGTGTCATTCTTGAAATTTCATGTTATAGT
     1450      1460      1470      1480      1490      1500

  D  T  V  S  E  S  S  S  Y  S  Y  G  E  I  P  F  G  I  T  D
 T  Q  *  V  S  L  V  L  T  V  M  V  K  S  R  S  A  *  L  T
   H  S  E  *  V  *  F  L  Q  L  W  *  N  P  V  R  H  N  *  R
GACACAGTGAGTGAGTCTAGTTCTTACAGTTATGGTGAAATCCCGTTCGGCATAACTGAC
     1510      1520      1530      1540      1550      1560

  G  P  R  Y  C  Y  V  L  Y  N  G  T  A  L  K  Y  L  G  T  L
 D  H  D  T  V  M  Y  F  T  M  A  Q  L  L  N  I  *  E  H  Y
   T  T  I  L  L  C  T  L  Q  W  H  S  S  *  I  F  R  N  I  T
GGACCACGATACTGTTATGTACTTTACAATGGCACAGCTCTTAAATATTTAGGAACATTA
     1570      1580      1590      1600      1610      1620

  P  P  S  V  K  E  I  A  I  S  K  W  G  H  F  Y  I  N  G  Y
 H  P  V  *  R  K  L  L  L  V  S  G  A  I  F  I  L  M  V  T
   T  Q  C  K  G  N  C  Y  *  *  V  G  P  F  L  Y  *  W  L  Q
CCACCCAGTGTAAAGGAAATTGCTATTAGTAAGTGGGGCCATTTTTATATTAATGGTTAC
     1630      1640      1650      1660      1670      1680

  N  F  F  S  T  F  P  I  G  C  I  S  F  N  L  T  T  G  V  S
 I  S  L  A  H  F  L  L  V  V  Y  L  L  I  *  P  L  V  L  V
   F  L  *  H  I  Y  L  Y  I  F  F  H  W  C  *  W
AATTTCTTTAGCACATTTCCTATTGGTTGTATATCTTTTAATTTAACCACTGGTGTTAGT
     1690      1700      1710      1720      1730      1740

  G  A  F  W  T  I  A  Y  T  S  Y  T  E  A  L  V  Q  V  E  N
 E  L  F  G  Q  L  L  T  H  R  I  L  K  H  *  Y  K  L  K  T
   S  F  L  D  N  C  L  H  I  V  Y  *  S  I  S  T  S  *  K  H
GGAGCTTTTTGGACAATTGCTTACACATCGTATACTGAAGCATTAGTACAAGTTGAAAAC
     1750      1760      1770      1780      1790      1800

  T  A  I  P  N  V  T  Y  C  N  S  H  I  N  N  I  K  C  S  Q
 Q  L  L  K  M  *  R  I  V  T  V  T  L  I  T  L  N  V  L  N
   S  Y  *  K  C  D  V  L  *  Q  S  H  *  *  H  *  M  F  S  T
ACAGCTATTAAAAATGTGACGTATTGTAACAGTCACATTAATAACATTAAATGTTCTCAA
     1810      1820      1830      1840      1850      1860

  L  T  A  N  N  N  G  F  Y  P  V  A  S  S  E  V  G  F  V
 L  L  L  I  *  I  M  D  F  I  L  L  L  Q  V  K  *  V  S  L
   Y  C  *  F  E  *  W  I  L  S  L  C  F  K  *  S  R  F  R  *
CTTACTGCTAATTTGAATAATGGATTTTATCCTGTTGCTTCAAGTGAAGTAGGTTTCGTT
     1870      1880      1890      1900      1910      1920
```

```
  N  K  S  V  V  L  L  P  S  F  F  T  Y  T  A  V  N  I  T  I
     V  L  C  Y  Y  L  A  F  S  H  T  P  L  S  I  *  P  L
           V  I  T  *  L  F  H  I  H  R  C  Q  Y  N  H  *
AATAAGAGTGTTGTGTTATTACC      TTTCACATACACCGCTGTCAATATAACCATT
    1930      1940      1950           1970      1980


  D  L  G  M  K  L  S  G  Y  G  Q  P  I  A  S     S  N  I
     I  L  V  *  S  L  V  V  M  V  N  P  *  P  R  H  *  V  T  S
     S  W  Y  E  A  *  W  L  W  S  T  H  S  L  D  T  K  *  H  H
GATCTTGGTATGAAGCTTAGTGGTTATGGTCAACCCATAGCCTCGACACTAAGTAACATC
    1990      2000      2010      2020      2030      2040


  T  L  P  M  Q  D  N  N  T  D  V  Y  C  I  R  S  N  Q  F  S
     H  Y  Q  C  R  I  T  I  L  M  C  T  V  F  V  L  T  N  S  Q
     T  T  N  A  G  *  Q  Y  *  C  V  L  Y  S  F  *  P  I  L  S
ACACTACCAATGCAGGATAACAATACTGATGTGTACTGTATTCGTTCTAACCAATTCTCA
    2050      2060      2070      2080      2090      2100


  V  Y  V  H  S  T  C  K  S  S  L  W  D  N  I  F  N  Q  D  C
     F  M  F  I  P  L  A  K  V  L  Y  G  T  I  F  L  I  K  T  A
     L  C  S  F  H  L  Q  K  F  F  M  G  Q  Y  F  *  S  R  L  H
GTTTATGTTCATTCCACTTGCAAAAGTTCTTTATGGGACAATATTTTTAATCAAGACTGC
    2110      2120      2130      2140      2150      2160


  T  D  V  L  E  A  T  A  V  I  K  T  G  T  C  P  F  S  F  D
     R  M  F  *  R  L  Q  L  L  *  K  L  V  L  V  L  S  H  L  I
     G  C  F  R  G  Y  S  C  Y  K  N  W  Y  L  S  F  L  I  *  *
ACGGATGTTTTAGAGGCTACAGCTGTTATAAAAACTGGTACTTGTCCTTTCTCATTTGAT
    2170      2180      2190      2200      2210      2220


  K  L  N  N  Y  L  T  F  N  K  F  C  L  S  L  S  P  V  G  A
     N  *  T  I  T  *  L  L  T  S  S  V  C  R  *  V  L  L  V  L
     I  E  Q  L  L  D  F  *  Q  V  L  F  V  V  E  S  C  W  C  *
AAATTGAACAATTACTTGACTTTTAACAAGTTCTGTTTGTCGTTGAGTCCTGTTGGTGCT
    2230      2240      2250      2260      2270      2280


  N  C  K  F  D  V  A  A  R  T  R  T  N  E  Q  V  V  R  S  L
     I  A  S  L  M  L  L  H  V  Q  E  P  M  S  R  L  L  E  V  Y
     L  Q  V  *  C  C  C  T  Y  K  N  Q  *  A  G  C  *  K  S  I
AATTGCAAGTTTGATGTTGCTGCACGTACAAGAACCAATGAGCAGGTTGTTAGAAGTCTA
    2290      2300      2310      2320      2330      2340


  Y  V  I  Y  E  E  G  D  N  I  V  G  V  P  S  D  N  S  G  L
     M  *  Y  M  K  K  E  T  T  *  W  V  Y  R  L  I  I  A  V  C
     C  N  I  *  R  R  R  Q  H  S  G  C  T  V  *  *  *  R  S  A
TATGTAATATATGAAGAAGGAGACAACATAGTGGGTGTACCGTCTGATAATAGCGGTCTG
    2350      2360      2370      2380      2390      2400
```

```
   H  D  L  S  V  L  H  L  D  S  C  T  D  Y  N  I  Y  G  R  T
    T  I  C  L  C  Y  T  *  T  P  V  Q  I  T  I  Y  M  V  E  L
     R  F  V  C  A  T  P  R  L  L  Y  R  L  G  Y  I  W  *  N  W
  CACGATTTGTCTGTGCTACACCTAGACTCCTGTACAGATTACAATATATATGGTAGAACT
     2410      2420      2430      2440      2450      2460


   G  V  G  I  I  R  R  T  N  S  T  L  L  S  G  L  Y  Y  T  S
    V  L  V  L  L  D  E  L  T  V  R  Y  L  V  A  Y  I  T  H  H
     C  W  Y  Y  *  T  N  *  Q  Y  A  T  *  W  L  I  L  H  I  T
  GGTGTTGGTATTATTAGACGAACTAACAGTACGCTACTTAGTGGCTTATATTACACATCA
     2470      2480      2490      2500      2510      2520


   L  S  G  D  L  L  G  F  K  N  V  S  D  G  V  I  Y  S  V  T
    Y  Q  V  I  C  *  A  L  K  M  L  V  M  V  S  F  I  L  *  R
     I  R  *  F  V  R  L  *  K  C  *  *  W  C  H  L  F  C  D  A
  CTATCAGGTGATTTGTTAGGCTTTAAAAATGTTAGTGATGGTGTCATTTATTCTGTGACG
     2530      2540      2550      2560      2570      2580


   P  C  D  V  S  A  Q  A  A  V  I  D  G  A  I  V  G  A  M  T
    H  V  M  *..A  H  K  R  L  L  L  M  V  P  *  L  E  L  *  L
     M  *  C  K  R  T  S  G  C  Y  *  W  C  H  S  W  S  Y  D  F
  CCATGTGATGTAAGCGCACAAGCGGCTGTTATTGATGGTGCCATAGTTGGAGCTATGACT
     2590      2600      2610      2620      2630      2640


   S  I  N  S  E  L  L  G  L  T  H  W  T  T  T  P  N  F  Y  Y
    P  L  T  V  N  C  *  V  *  H  I  G  Q  R  H  L  I  F  I  T
     H  *  Q  *  T  V  R  S  N  T  L  D  N  D  T  *  F  L  L  L
  TCCATTAACAGTGAACTGTTAGGTCTAACACATTGGACAACGACACCTAATTTTTATTAC
     2650      2660      2670      2680      2690      2700


   Y  S  I  Y  N  Y  T  S  E  R  T  R  G  T  A  I  D  S  N  D
    T  L  Y  I  I  T  Q  V  R  G  L  V  A  L  Q  L  T  V  T  M
     L  Y  I  *  L  H  K  *  E  D  S  W  H  C  N  *  Q  *  R  C
  TACTCTATATATAATTACACAAGTGAGAGGACTCGTGGCACTGCAATTGACAGTAACGAT
     2710      2720      2730      2740      2750      2760


   V  D  C  E  P  V  I  T  Y  S  N  I  G  V  C  K  N  G  A  L
    L  I  V  N  L  S  *  P  I  L  I  *  V  F  V  K  M  V  L  W
     *  L  *  T  C  H  N  L  F  *  Y  R  C  L  *  K  W  C  F  G
  GTTGATTGTGAACCTGTCATAACCTATTCTAATATAGGTGTTTGTAAAAATGGTGCTTTG
     2770      2780      2790      2800      2810      2820


   V  F  I  N  V  T  H  S  D  G  D  V  Q  P  I  S  T  G  N  V
    F  L  L  T  S  H  I  L  T  E  T  C  N  Q  L  A  L  V  M  S
     F  Y  *  R  H  T  F  *  R  R  R  A  T  N  *  H  W  *  C  H
  GTTTTTATTAACGTCACACATTCTGACGGAGACGTGCAACCAATTAGCACTGGTAATGTC
     2830      2840      2850      2860      2870      2880
```

DUPHAR INTERNATIONAL RESEARCH B.V.

```
  T  I  P  T  N  F  T  I  S  V  Q  V  E  Y  M  Q  V  Y  T  T
   R  Y  L  Q  I  L  L  Y  L  C  K  L  N  T  C  R  F  T  L  H
    D  T  Y  K  F  Y  Y  I  C  A  S  *  I  H  A  G  L  H  Y  T
ACGATACCTACAAATTTTACTATATCTGTGCAAGTTGAATACATGCAGGTTTACACTACA
     2890      2900      2910      2920      2930      2940


  P  V  S  I  D  C  A  R  Y  V  C  N  G  N  P  R  C  N  K  L
   Q  Y  Q  *  I  V  Q  D  T  F  V  M  V  T  L  D  V  T  N  C
    S  I  N  R  L  C  K  I  R  L  *  W  *  P  *  M  *  Q  I  V
CCAGTATCAATAGATTGTGCAAGATACGTTTGTAATGGTAACCCTAGATGTAACAAATTG
     2950      2960      2970      2980      2990      3000


  L  T  Q  Y  V  S  A  C  Q  T  I  E  Q  A  L  A  M  G  A  R
   *  H  N  M  C  L  H  V  K  L  L  N  K  H  L  Q  W  V  P  D
    N  T  I  C  V  C  M  S  N  Y  *  T  S  T  C  N  G  C  Q  T
TTAACACAATATGTGTCTGCATGTCAAACTATTGAACAAGCACTTGCAATGGGTGCCAGA
     3010      3020      3030      3040      3050      3060


  L  E  N  M  E  V  D  S  M  L  F  V  S  E  N  A  L  K  L  A
   L  K  T  W  R  L  I  P  C  C  L  S  R  K  M  P  L  N  W  H
    *  K  H  G  G  *  F  H  V  V  C  L  G  K  C  P  *  I  G  I
CTTGAAAACATGGAGGTTGATTCCATGTTGTTTGTCTCGGAAAATGCCCTTAAATTGGCA
    -3070      3080      3090       3100      3110      3120


  S  V  E  A  F  N  S  T  E  N  L  D  P  I  Y  K  E  W  P  S
   L  L  R  R  S  I  V  Q  K  I  *  I  L  F  T  K  N  G  L  A
    C  *  G  V  Q  *  Y  R  K  F  R  S  Y  L  Q  R  M  A  *  H
TCTGTTGAGGCGTTCAATAGTACAGAAAATTTAGATCCTATTTACAAAGAATGGCCTAGC
      3130      3140       3150       3160      3170      3180


  I  G  G  S  W  L  G  G  L  K  D  I  L  P  S  H  N  S  K  R
   *  V  V  L  G  *  E  V  *  K  I  Y  Y  R  P  I  I  A  N  V
    R  W  F  L  A  R  R  S  K  R  Y  T  T  V  P  *  *  Q  T  *
ATAGGTGGTTCTTGGCTAGGAGGTCTAAAAGATATACTACCGTCCCATAATAGCAAACGT
     3190      3200      3210      3220      3230      3240


  K  Y  G  S  A  I  E  D  L  L  F  D  K  V  V  T  S  G  L  G
   S  M  V  L  L  *  K  I  C  F  L  I  K  L  *  H  L  V  *  V
    V  W  F  C  Y  R  R  F  A  F  *  *  S  C  N  I  W  F  R  Y
AAGTATGGTTCTGCTATAGAAGATTTGCTTTTTGATAAAGTTGTAACATCTGGTTTAGGT
     3250      3260      3270      3280      3290      3300


  T  V  D  E  D  Y  K  R  C  T  G  G  Y  D  I  A  D  L  V  C
   Q  L  M  K  I  I  N  V  V  L  V  V  T  T  *  Q  T  W  C  V
    S  *  *  R  L  *  T  L  Y  W  W  L  R  H  S  R  L  G  V  C
ACAGTTGATGAAGATTATAAACGTTGTACTGGTGGTTACGACATAGCAGACTTGGTGTGT
     3310      3320      3330      3340      3350      3360
```

```
       A   Q  Y  Y  N  G  I  M  V  L  P  G  V  A  N  A  D  K  M  T
       L   N  I  T  M  A  S  W  F  Y  Q  V  *  L  M  L  T  R  *  L
        S  I  L  Q  W  H  H  G  S  T  R  C  S  *  C  *  Q  D  D  Y
       GCTCAATATTACAATGGCATCATGGTTCTACCAGGTGTAGCTAATGCTGACAAGATGACT
           3370      3380      3390      3400      3410      3420


     M  Y  T  A  S  L  A  G  G  I  T  L  G  A  L  G  G  G  A  V
       C  T  Q  H  H  L  Q  V  V  *  H  *  V  H  L  V  V  A  P  W
        V  H  S  I  T  C  R  W  Y  N  I  R  C  T  W  W  W  R  R  G
       ATGTACACAGCATCACTTGCAGGTGGTATAACATTAGGTGCACTTGGTGGTGGCGCCGTG
           3430      3440      3450      3460      3470      3480


       A  I  P  F  A  V  A  V  Q  A  R  L  N  Y  V  A  L  Q  T  D
        L  Y  L  L  Q  *  Q  Y  R  L  D  L  I  M  L  L  Y  K  L  M
         Y  T  F  C  S  S  S  T  G  *  T  *  L  C  C  S  T  N  *  C
       GCTATACCTTTTGCAGTAGCAGTACAGGCTAGACTTAATTATGTTGCTCTACAAACTGAT
           3490      3500      3510      3520      3530      3540


       V  L  N  K  N  Q  Q  I  L  A  N  A  F  N  Q  A  I  G  N  I
        Y  *  I  K  T  N  R  S  W  L  M  L  S  I  K  L  L  V  T  L
         I  E  *  K  P  T  D  P  G  *  C  F  Q  S  S  Y  W  *  H  Y
       GTATTGAATAAAAACCAACAGATCCTGGCTAATGCTTTCAATCAAGCTATTGGTAACATT
           3550      3560      3570      3580      3590      3600


       T  Q  A  F  G  K  V  N  D  A  I  H  Q  T  S  Q  G  L  A  T
        H  R  L  L  V  R  L  M  M  L  Y  I  K  H  H  K  V  L  P  L
         T  G  F  W  *  G  *  *  C  Y  T  S  N  I  T  R  S  C  H  C
       ACACAGGCTTTTGGTAAGGTTAATGATGCTATACATCAAACATCACAAGGTCTTGCCACT
           3610      3620      3630      3640      3650      3660


       V  A  K  A  L  A  K  V  Q  D  V  V  N  T  Q  G  Q  A  L  S
        L  L  K  R  W  Q  K  C  K  M  L  S  T  H  K  G  K  L  *  V
         C  *  S  V  G  K  S  A  R  C  C  Q  H  T  R  A  S  F  K  S
       GTTGCTAAAGCGTTGGCAAAAGTGCAAGATGTTGTCAACACACAAGGGCAAGCTTTAAGT
           3670      3680      3690      3700      3710      3720


       H  L  T  V  Q  L  Q  N  N  F  Q  A  I  S  S  S  I  S  D  I
        T  L  Q  Y  N  C  K  I  I  F  K  P  L  V  V  L  L  V  I  F
         P  Y  S  T  I  A  K  *  F  S  S  H  *  *  F  Y  *  *  Y  L
       CACCTTACAGTACAATTGCAAAATAATTTTCAAGCCATTAGTAGTTCTATTAGTGATATT
           3730      3740      3750      3760      3770      3780


       Y  N  R  L  D  E  L  S  A  D  A  Q  V  D  R  L  I  T  G  R
        I  T  G  L  T  N  *  V  L  M  H  K  L  I  G  *  L  Q  V  D
         *  Q  A  *  R  T  E  C  *  C  T  S  *  *  A  D  Y  R  *  T
       TATAACAGGCTTGACGAACTGAGTGCTGATGCACAAGTTGATAGGCTGATTACAGGTAGA
           3790      3800      3810      3820      3830      3840
```

```
L  T  A  L  N  A  F  V  S  Q  T  L  T  R  Q  A  E  V  R  A
 L  Q  H  L  M  H  L  C  L  R  L  *  P  D  K  Q  R  L  G  L
  Y  S  T  *  C  I  C  V  S  D  S  N  Q  T  S  R  G  *  G  *
CTTACAGCACTTAATGCATTTGTGTCTCAGACTCTAACCAGACAAGCAGAGGTTAGGGCT
     3850      3860      3870      3880      3890      3900


 S  R  Q  L  A  K  D  K  V  N  E  C  V  R  S  Q  S  Q  R  F
  V  D  N  L  P  K  T  R  L  M  N  V  L  G  L  S  L  R  D  S
   *  T  T  C  Q  R  Q  G  *  *  M  C  *  V  S  V  S  E  I  R
AGTAGACAACTTGCCAAAGACAAGGTTAATGAATGTGTTAGGTCTCAGTCTCAGAGATTC
     3910      3920      3930      3940      3950      3960


 G  F  C  G  N  G  T  H  L  F  S  L  A  N  A  A  P  N  G  M
  D  S  V  V  M  V  H  I  C  F  H  *  Q  M  Q  H  Q  M  A  *
   I  L  W  *  W  Y  T  F  V  F  T  S  K  C  S  T  K  W  H  D
GGATTCTGTGGTAATGGTACACATTTGTTTTCACTAGCAAATGCAGCACCAAATGGCATG
     3970      3980      3990      4000      4010      4020


 I  F  F  H  T  V  L  L  P  T  A  Y  E  T  V  T  A  W  S  G
  F  S  F  I  Q  Y  Y  Y  Q  Q  L  M  K  L  *  Q  L  G  Q  V
   F  L  S  Y  S  T  I  T  N  S  L  *  N  C  N  S  L  V  R  Y
ATTTTCTTTCATACAGTACTATTACCAACAGCTTATGAAACTGTAACAGCTTGGTCAGGT
     4030      4040      4050      4060      4070      4080


 I  C  A  S  D  G  D  R  T  F  G  L  V  V  K  D  V  Q  L  T
  F  V  L  Q  M  A  I  A  L  S  D  L  S  L  K  M  C  S  *  R
   L  C  F  R  W  R  S  H  F  R  T  C  R  *  R  C  A  V  D  V
ATTTGTGCTTCAGATGGCGATCGCACTTTCGGACTTGTCGTTAAAGATGTGCAGTTGACG
     4090      4100      4110      4120      4130      4140


 L  F  R  N  L  D  D  K  F  Y  L  T  P  R  T  M  Y  Q  P  R
  C  F  V  I  *  M  T  S  S  I  *  P  P  E  L  C  I  S  L  E
   V  S  *  S  R  *  Q  V  L  F  D  P  Q  N  Y  V  S  A  *  S
TTGTTTCGTAATCTAGATGACAAGTTCTATTTGACCCCCAGAACTATGTATCAGCCTAGA
     4150      4160      4170      4180      4190      4200


 V  A  T  S  S  D  F  V  Q  I  E  G  C  D  V  L  F  V  N  A
  L  Q  L  V  L  I  L  F  K  L  K  G  V  M  C  C  L  S  T  R
   C  N  *  F  *  F  C  S  N  *  R  V  *  C  V  V  C  Q  R  D
GTTGAACTAGTTCTGATTTTGTTCAAATTGAAGGGTGTGATGTGTTGTTTGTCAACGCG
     4210      4220      4230      4240      4250      4260


 T  V  I  D  L  P  S  I  I  P  D  Y  I  D  I  N  Q  T  V  Q
  L  *  L  I  C  L  V  L  Y  L  T  I  L  T  L  I  K  L  F  K
   C  N  *  F  A  *  Y  Y  T  *  L  Y  *  H  *  S  N  C  S  R
ACTGTAATTGATTTGCCTAGTATTATACCTGACTATATTGACATTAATCAAACTGTTCAA
     4270      4280      4290      4300      4310      4320
```

DUPHAR INTERNATIONAL RESEARCH B.V.

```
     D   I   L   E   N   Y   R   P   N   W   T   V   P   E   F   T   L   D   I   F
       T   Y   *   K   I   T   D   Q   T   G   L   Y   L   N   L   H   L   I   F   S
         H   I   R   K   L   Q   T   K   L   D   C   T   *   I   Y   T   *   Y   F   Q
     GACATATTAGAAAATTACAGACCAAACTGGACTGTACCTGAATTTACACTTGATATTTTC
          4330        4340        4350        4360        4370        4380


     N   A   T   Y   L   N   L   T   G   E   I   D   D   L   E   F   R   S   E   K
       T   Q   P   I   *   I   *   L   V   K   L   M   T   *   S   L   G   Q   K   S
         R   N   L   F   K   S   D   W   *   N   *   *   L   R   V   *   V   R   K   A
     AACGCAACCTATTTAAATCTGACTGGTGAAATTGATGACTTAGAGTTTAGGTCAGAAAAG
          4390        4400        4410        4420        4430        4440


     L   H   N   T   T   V   E   L   A   I   L   I   D   N   I   N   N   T   L   V
       Y   I   T   L   Q   *   N   L   P   F   S   L   I   T   L   I   I   H   *   S
         T   *   H   Y   S   R   T   C   H   S   H   *   *   H   *   *   Y   I   S   Q
     CTACATAACACTACAGTAGAACTTGCCATTCTCATTGATAACATTAATAATACATTAGTC
          4450        4460        4470        4480        4490        4500


     N   L   E   W   L   N   R   I   E   T   Y   V   K   W   P   W   Y   V   W   L
       I   L   N   G   S   I   E   L   K   L   M   *   N   G   L   G   M   C   G   Y
         S   *   M   A   Q   *   N   *   N   L   C   K   M   A   L   V   C   V   A   T
     AATCTTGAATGGCTCAATAGAATTGAAACTTATGTAAAATGGCCTTGGTATGTGTGGCTA
          4510        4520        4530        4540        4550        4560


     L   I   G   L   V   V   V   F   C   I   P   L   L   L   F   C   C   F   S   T
       *   *   V   *   *   *   Y   F   A   Y   H   Y   C   Y   F   A   V   L   A   Q
         D   R   F   S   S   S   I   L   H   T   I   T   A   I   L   L   F   *   H   R
     CTGATAGGTTTAGTAGTAGTATTTTGCATACCATTACTGCTATTTTGCTGTTTTAGCACA
          4570        4580        4590        4600        4610        4620


     G   C   C   G   C   I   G   C   L   G   S   C   C   H   S   I   C   S   R   R
       V   V   V   D   A   *   V   V   *   E   V   V   V   T   L   Y   V   V   E   D
         L   L   W   M   H   R   L   F   R   K   L   L   S   L   Y   M   *   *   K   T
     GGTTGTTGTGGATGCATAGGTTGTTTAGGAAGTTGTTGTCACTCTATATGTAGTAGAAGA
          4630        4640        4650        4660        4670        4680


     Q   F   E   N   Y   E   P   I   E   K   V   H   V   H   *   I   *   S   *   G
       N   L   K   I   M   N   Q   L   K   K   C   M   S   T   K   F   K   V   K   D
         I   *   K   L   *   T   N   *   K   S   A   C   P   L   N   L   K   L   R   M
     CAATTTGAAAATTATGAACCAATTGAAAAAGTGCATGTCCACTAAATTTAAAGTTAAGGA
          4690        4700        4710        4720    ↑   4730        4740


     C   *   I   N   S   L   R   T   K   L   I   S   H   Y   R   S   C   M   D   I
       V   E   *   I   P   *   E   L   N   L   L   V   I   T   G   L   V   W   T   L
         L   N   K   F   L   K   N   *   T   Y   *   S   L   Q   V   L   Y   G   H   C
     TGTTGAATAAATTCCTTAAGAACTAAACTTATTAGTCATTACAGGTCTTGTATGGACATT
          4750        4760        4770        4780        4790        4800
```

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | JOURNAL OF GENERAL VIROLOGY, vol. 66, no. 4, April 1985, pages 719-726, SGM, GB; M.M. BINNS et al.: "Cloning and sequencing of the gene encoding the spike protein of the coronavirus IBV" * Whole article * | 1,5-9 | C 12 N 15/00 A 61 K 39/215 A 61 K 37/02 C 07 K 13/00 |
| A | JOURNAL OF GENERAL VIROLOGY, vol. 64, no. 9, September 1983, pages 1859-1866, SGM, GB; H.E.L. JACOBSE-GEELS et al.: "Expression of feline infectious peritonitis coronavirus antigens on the surface of feline macrophage-like cells" * Pages 1862,1863 * | 2,3 | |
| A | EP-A-0 138 242 (DUPHAR INTERNATIONAL RESEARCH B.V.) | 1-9 | |

TECHNICAL FIELDS
SEARCHED (Int. Cl.4)

C 12 N
A 61 K

The present search report has been drawn up for all claims

| Place of search | Date of completion of the search | Examiner |
|---|---|---|
| THE HAGUE | 15-01-1988 | CUPIDO M. |